# EUROPEAN PATENT SPECIFICATION

(11) **EP 3 435 990 B1**
(45) Date of publication and mention of the grant of the patent: **27.01.2021**
(21) Application number: 17773436.5
(22) Date of filing: 28.03.2017
(51) Int. Cl.: A61K 9/06, A61K 47/06, A61K 47/14, A61K 31/47, A61K 47/20, A61P 17/00, A61P 17/06, A61P 35/00

(54) **STABLE PHARMACEUTICAL COMPOSITIONS FOR TOPICAL ADMINISTRATION AND USES THEREOF**
STABILE PHARMAZEUTISCHE ZUSAMMENSETZUNGEN ZUR TOPISCHEN VERABREICHUNG UND VERWENDUNGEN DAVON
COMPOSITIONS PHARMACEUTIQUES STABLES POUR ADMINISTRATION TOPIQUE ET LEURS UTILISATIONS

(30) Priority: 28.03.2016 US 201662313836 P
(43) Date of publication of application: 06.02.2019
(73) Proprietor: VIDAC Pharma Ltd., 9777510 Jerusalem (IL)
(72) Inventor: SALAMA, Paul, Ashdod (IL); BECKER, Oren Menahem, 9074811 Mevasseret Zion (IL); MANN BRICKMAN, Chaim, 4322714 Ra'anana (IL)
(74) Representative: Pearl Cohen Zedek Latzer Baratz UK LLP
(86) International application number: PCT/IL2017/050381
(87) International publication number: WO 2017/168416

(56) References cited:
- WO-A1-2004/012716
- WO-A1-2008/007367
- WO-A2-2007/066336
- WO-A2-2007/066336
- US-A1- 2015 080 430

## Description

### FIELD OF THE INVENTION

The present invention provides stable topical pharmaceutical compositions comprising a compound of Formula I (Compound I), and the use of these compositions for treating or preventing cancers or pre-cancerous conditions, or benign hyperproliferative disorders, such as actinic keratosis.

### BACKGROUND OF THE INVENTION

Jasmonates are a family of plant stress hormones, which are released in instances of extreme UV radiation, osmotic shock, heat shock, pathogen attack and the like, to initiate various cascades. The use of jasmonates for the treatment of mammalian cancer has been disclosed in International Patent Application WO 02/080890 and in U.S. Patent No. 6,469,061 wherein the jasmonates were shown to induce direct cytotoxicity for various types of human cancer cells derived from breast, prostate, skin, and blood cancers. Methyl jasmonate was shown to be effective in preventing development of lymphomas in mice (Fingrut and Flescher, Leukemia, 16: 608-616, 2002).

International Patent Application WO 2005/054172 discloses halogenated jasmonate derivatives, pharmaceutical compositions comprising the derivatives, and their use in reducing cancer cell growth and in treating cancer.

International Patent Applications WO 2007/066336 and WO 2007/066337 disclose jasmonate derivatives, pharmaceutical compositions comprising same, and use thereof in reducing cancer cell growth and the treatment of cancer.

International Patent Applications WO 2010/143180 discloses jasmonate derivatives, an assay for determining the therapeutic effect of a jasmonate ester derivative in benign hyperproliferative skin disorders, pharmaceutical compositions comprising jasmonate derivatives, and use thereof in treating benign hyperproliferative disorders of the skin, in particular, actinic keratosis.

Compounds comprising an ester group are prone to hydrolysis, which constitutes an obstacle in the preparation of a suitable formulation which maintains the structural integrity of the active compound. There is an unmet need for stable pharmaceutical compositions comprising such compounds, especially a compound of Formula I as described herein. Such compounds are useful for the treatment or prevention of cancer or pre-cancerous conditions, or benign hyperproliferative disorders.

### SUMMARY OF THE INVENTION

The present invention provides novel formulations for topical administration of a compound represented by the structure of Formula I (Compound I), and pharmaceutically acceptable excipients.

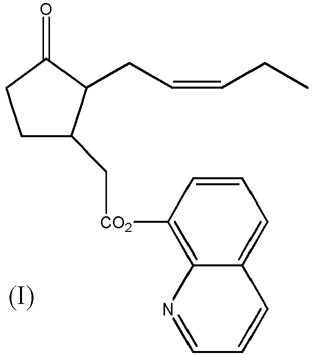

The formulations of the invention are specifically designed to stabilize Compound I, which contains an ester group that is prone to lysis in nucleophilic media (e.g., water, alcohols). As demonstrated herein, conventionally used topical excipients could not preserve the stability of Compound I and demonstrated poor chemical compatibility and degradation of the active ester component. It has now been surprisingly discovered that a specific combination of excipients can provide stable formulations containing Compound I for an improved therapeutic benefit by topical administration. These excipients are devoid of hydroxyl (OH) or other nucleophilic functionalities, and are mainly non-polar compounds that are compatible with the lipophilic nature of Compound I.

Thus, the present invention provides, in one aspect, a topical pharmaceutical composition comprising a therapeutically effective amount of a compound represented by the structure of Formula I, or a pharmaceutically acceptable salt or solvate thereof, and at least one pharmaceutically acceptable excipient comprising a glycol diester with a saturated or unsaturated fatty acid, a triglyceride and an aprotic organic solvent. In one embodiment, at least one of the excipients is a non-aqueous excipient. Optionally, the composition further comprises an oil, a wax, or a combination thereof.

In one embodiment, the triglyceride comprises a compound represented by the structure: 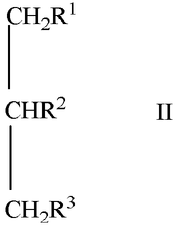 wherein:
R¹, R² and R³ are each R⁴-COO-; and
R⁴-COO- is the residue of a saturated or unsaturated fatty acid.

In one embodiment, the pharmaceutically acceptable excipient comprises a triglyceride. In some embodiments, the triglyceride comprises medium chain (e.g., C₈-C₁₂) saturated or unsaturated fatty acids. In one embodiment, the pharmaceutically acceptable excipient is caprylic/capric triglyceride (in one embodiment, Labrafac lipophlie WL 1349).

In another embodiment, the pharmaceutically acceptable excipient comprises a glycol diester. In some embodiments, the glycol diester is a propylene glycol diester with saturated or unsaturated fatty acids. In one embodiment, the fatty acid is a medium chain (e.g., C₈-C₁₂) saturated or unsaturated fatty acid. In one embodiment, the pharmaceutically acceptable excipient is propylene glycol dicaprylate (in one embodiment, Labrafac PG).

In some embodiments, the pharmaceutically acceptable excipient comprises a mixture of a triglyceride and a propylene glycol diester with saturated or unsaturated fatty acids.

In one embodiment, the pharmaceutically acceptable excipient is an organic solvent, which, in one embodiment, is dimethylsulfoxide (DMSO).

In another embodiment, the composition of the invention further comprises an oil, a wax or a combination thereof.

In some embodiments, the composition of the invention comprises the compound of Formula I, propylene glycol dicaprylate, caprylic/capric triglyceride, dimethylsulfoxide (DMSO), petrolatum and paraffin wax.

In some embodiments, the composition of the invention comprises a compound of Formula I in an amount of from about 5% w/w to about 30% w/w. In some embodiments, the composition of the invention comprises a compound of Formula I in an amount of about 5% w/w. In some embodiments, the composition of the invention comprises a compound of Formula I in an amount of about 10% w/w. In some embodiments, the composition of the invention comprises a compound of Formula I in an amount of about 15% w/w. In some embodiments, the composition of the invention comprises a compound of Formula I in an amount of about 20% w/w.

In some embodiments, the composition of the invention comprises: a compound of Formula I in an amount of from about 5% to about 30% w/w; propylene glycol dicaprylate in an amount of from about 1% to about 10% w/w; caprylic/capric triglyceride in an amount of from about 30% to about 50% w/w; dimethyl sulfoxide in an amount of from about 0% to about 10% w/w; petrolatum in an amount of from about 20% to about 50% w/w; and paraffin wax in an amount of from about 0 to about 10% w/w. The term "w/w" denotes weight/weight.

In other embodiments, the composition of the invention comprises: a compound of Formula I in an amount of about 5% w/w; propylene glycol dicaprylate in an amount of about 10% w/w; caprylic/capric triglyceride in an amount of about 50% w/w; dimethyl sulfoxide in an amount of about 1% w/w; petrolatum in an amount of about 30% w/w; and paraffin wax in an amount of about 4% w/w.

In other embodiments, the composition of the invention comprises: a compound of Formula I in an amount of about 10% w/w; propylene glycol dicaprylate in an amount of about 7.5% w/w; caprylic/capric triglyceride in an amount of about 47.5% w/w; dimethyl sulfoxide in an amount of about 1% w/w; petrolatum in an amount of about 30% w/w; and paraffin wax in an amount of about 4% w/w.

In other embodiments, the composition of the invention comprises: a compound of Formula I in an amount of about 15% w/w; propylene glycol dicaprylate in an amount of about 5% w/w; caprylic/capric triglyceride in an amount of about 45% w/w; dimethyl sulfoxide in an amount of about 1% w/w; petrolatum in an amount of about 30% w/w; and paraffin wax in an amount of about 4% w/w.

In other embodiments, the composition of the invention comprises: a compound of Formula I in an amount of about 20% w/w; propylene glycol dicaprylate in an amount of about 2.5% w/w; caprylic/capric triglyceride in an amount of about 42.5% w/w; dimethyl sulfoxide in an amount of about 1% w/w; petrolatum in an amount of about 30% w/w; and paraffin wax in an amount of about 4% w/w.

In some embodiments, the topical pharmaceutical compositions are formulated as an ointment, cream or gel. In one embodiment, the composition is in the form of an ointment.

In some embodiments, the composition described above is essentially free of water, alcoholic solvents and carboxylic acids.

In another aspect, the present invention further provides a composition as described above for use in treating or preventing cancer, a pre-cancerous condition or a benign hyperproliferative disorder.

In some embodiments, the composition as described herein is administered in the form of an ointment, cream or gel. In certain embodiments, the composition is administered in the form of an ointment. In some embodiments, the composition is administered one time per day. In other embodiments, the composition is administered 2, 3, or 4 times per day. In some embodiments, the composition is administered once every 2 days, or once every 3 days, or once every 4 days, or once every 5 days, or once every 6 days, or once every 7 days, or once every 8 days, or once every 9 days, or once every 10 days. In other embodiments, the composition is administered once every week, or once every 2 weeks, or once every 3 weeks, or once every 4 weeks, or once every 5 weeks, or once every 6 weeks, or once every 7 weeks, or once every 8 weeks.

In one embodiment, the compound of Formula I is useful for treating actinic keratosis or psoriasis.

In some embodiments, the cancer is skin cancer selected from melanoma and non-melanoma skin cancer. In some embodiments, the non-melanoma skin cancer is selected from basal cell carcinoma (BCC) and squamous cell carcinoma (SCC).

In some embodiments, the composition can be administered in combination with at least one other active agent.

### DETAILED DESCRIPTION OF THE INVENTION

The present invention relates to compositions comprising a compound of Formula I and use thereof in treating or preventing cancer, pre-cancerous conditions, or benign hyperproliferative disorders.

The compound of Formula I comprises an ester moiety, which is prone to lysis upon contact with protic environment such as water and alcoholic solvents. Thus, many conventionally used excipients useful for topical formulations are not suitable for use in combination with Compound I as the latter is unstable and decomposes in their presence. The present invention provides uniquely designed formulations comprising pharmaceutical excipients that are suitable for use in topical compositions on the one hand, while preserving the stability of the compound of Formula I on the other. Such formulations contain excipients that are compatible with the lipophilic nature of Compound I. The formulations of the invention have utility for preventing cancer, pre-cancerous conditions, and benign hyperproliferative disorders.

In accordance with the principles of the present invention, Compound I is mixed with stabilizing excipients useful for topical administration in a formulation which is essentially free of compounds bearing a nucleophilic character like water, alcoholic solvents and carboxylic acids. In some embodiments, the topical pharmaceutical composition of the present invention comprises a therapeutically effective amount of a compound represented by the structure of Formula I, or a salt or solvate thereof, and at least one pharmaceutically acceptable excipient comprising a glycol diester with a saturated or unsaturated fatty acid, a triglyceride, an aprotic organic solvent, or any combination thereof, and optionally an oil and/or a wax

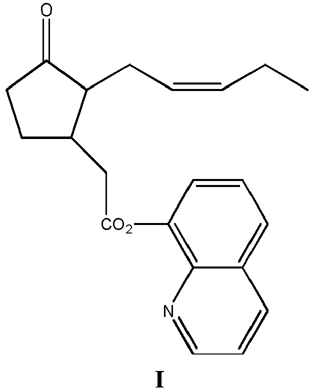

In one embodiment, at least one of the excipients is a non-aqueous excipient.

In accordance with the principles of the present invention, Compound I is mixed with stabilizing excipients useful for topical administration in a formulation which is essentially free of compounds bearing a nucleophilic character like water, alcoholic solvents and carboxylic acids. In some embodiments, the topical pharmaceutical composition of the present invention comprises a therapeutically effective amount of a compound represented by the structure of Formula I, or a pharmaceutically acceptable salt or solvate thereof, and at least one pharmaceutically acceptable excipient comprising a glycol diester with a saturated or unsaturated fatty acid, a triglyceride and an aprotic organic solvent, and optionally an oil and/or a wax

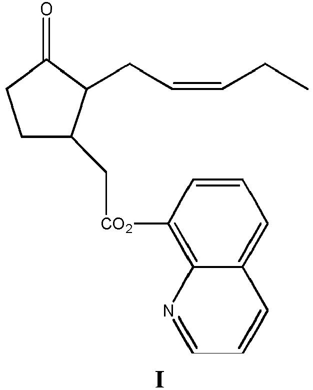

In one embodiment, the triglyceride comprises a compound represented by the structure: 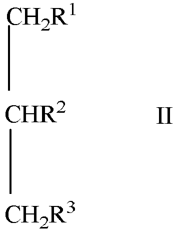 wherein:
R¹, R² and R³ are each R⁴-COO-; and
R⁴-COO- is the residue of a saturated or unsaturated fatty acid.

In another embodiment, the pharmaceutically acceptable excipient comprises a triglyceride of Formula III or a combination thereof 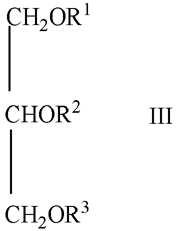 wherein
R¹, R² and R³ are each R⁴-C(=O)-; and R⁴-C(=O)O- is the residue of an unsaturated or saturated fatty acid.

In some embodiments, the pharmaceutically acceptable excipient comprises a triglyceride with a medium chain (e.g., C₈-C₁₂) saturated or unsaturated fatty acid. In one embodiment, the pharmaceutically acceptable excipient is caprylic/capric triglyceride (in one embodiment Labrafac lipophile WL 1349).

In another embodiment, the pharmaceutically acceptable excipient comprises a propylene glycol diester with a saturated or unsaturated fatty acid. In one embodiment, the fatty acid is a medium chain (e.g., C₈-C₁₂) saturated or unsaturated fatty acid. In one embodiment, the pharmaceutically acceptable excipient is propylene glycol dicaprylate (in one embodiment, Labrafac PG).

In one embodiment, the organic solvent is dimethylsulfoxide (DMSO).

The present invention may further comprise optional components such as oils or waxes so as to provide a semi-solid consistency as desired. Examples of such optional components are oils and/or waxes. Additional inactive excipients such as preservatives, anti-oxidants, perfumes and the like can also be added.

Oils that can be used as optional components in the compositions of the present invention include, but are not limited to, petrolatum (petroleum jelly), vegetable oil, fruit oil, plant oil, animal oil, mineral oil, coconut oil, olive oil, lanolin, peanut oil, hydrogenated and sulphated oils such as cotton seed oil, soybean oils, almond oil, sesame oil, and the like.

Waxes that can be used as optional components in the compositions of the present invention include, but are not limited to, paraffin wax, beeswax, carnauba wax, cetyl ester wax, microcrystalline wax, spermaceti wax, and the like.

In some embodiments, the composition of the invention comprises the compound represented by the structure of Formula I, propylene glycol dicaprylate (Labrafac PG), caprylic/capric triglyceride (Labrafac lipophile WL 1349), dimethylsulfoxide (DMSO), petrolatum and paraffin wax.

In some embodiments, the composition of the invention comprises a compound of Formula I in an amount of from about 5% w/w to about 30% w/w. In some embodiments, the composition of the invention comprises a compound of Formula I in an amount of about 5% w/w. In some embodiments, the composition of the invention comprises a compound of Formula I in an amount of about 10% w/w. In some embodiments, the composition of the invention comprises a compound of Formula I in an amount of about 15% w/w. In some embodiments, the composition of the invention comprises a compound of Formula I in an amount of about 20% w/w.

In some embodiments, the composition of the invention comprises a compound of Formula I in an amount of from about 5% to about 30% w/w; propylene glycol dicaprylate in an amount of from about 1% to about 10% w/w; caprylic/capric triglyceride in an amount of from about 30% to about 50% w/w; dimethyl sulfoxide in an amount of from about 0% to about 10% w/w; petrolatum in an amount of from about 20% to about 50% w/w; and paraffin wax in an amount of from about 0 to about 10% w/w.

In some embodiments, the composition of the invention comprises a compound of Formula I in an amount of about 5% w/w; propylene glycol dicaprylate in an amount of about 10% w/w; caprylic/capric triglyceride in an amount of about 50% w/w; dimethyl sulfoxide in an amount of about 1% w/w; petrolatum in an amount of about 30% w/w; and paraffin wax in an amount of about 4% w/w.

In other embodiments, the composition of the invention comprises a compound of Formula I in an amount of about 10% w/w; propylene glycol dicaprylate in an amount of about 7.5% w/w; caprylic/capric triglyceride in an amount of about 47.5% w/w; dimethyl sulfoxide in an amount of about 1% w/w; petrolatum in an amount of about 30% w/w; and paraffin wax in an amount of about 4% w/w.

In other embodiments, the composition of the invention comprises: a compound of Formula I in an amount of about 15% w/w; propylene glycol dicaprylate in an amount of about 5% w/w; caprylic/capric triglyceride in an amount of about 45% w/w; dimethyl sulfoxide in an amount of about 1% w/w; petrolatum in an amount of about 30% w/w; and paraffin wax in an amount of about 4% w/w.

In certain embodiments, the composition of the invention comprises a compound of Formula I in an amount of about 20% w/w; propylene glycol dicaprylate in an amount of about 2.5% w/w; caprylic/capric triglyceride in an amount of about 42.5% w/w; dimethyl sulfoxide in an amount of about 1% w/w; petrolatum in an amount of about 30% w/w; and paraffin wax in an amount of about 4% w/w.

In some embodiments, the topical pharmaceutical compositions are formulated as an ointment, cream or gel. In one embodiment, the composition is in the form of an ointment.

In some embodiments, the composition described above is essentially free of water, alcoholic solvents and carboxylic acids.

The pharmaceutically acceptable excipients according to the principles of the present invention may be a triglyceride, a glycol diester or a combination thereof. In some embodiments, the triglyceride and/or the glycol diester comprise saturated or unsaturated fatty acids. The term "saturated fatty acid" encompasses a carboxylic acid with an elongated aliphatic saturated chain. The term "unsaturated fatty acid" encompasses a carboxylic acid with an elongated aliphatic chain which have one or more double bonds between the carbon atoms. The length of the carbon chain may vary, but may generally be between 4 and 30 carbon atoms (C₄-C₃₀). In some embodiments, the triglyceride and/or glycol diester comprises medium chain (e.g., C₈-C₁₂) saturated or unsaturated fatty acids.

Compound I may be present in the form of a salt. The term "salt" encompasses both basic and acid addition salts, and include salts formed with the organic and inorganic anions and cations discussed below. Examples of acid addition salts include hydrochloric, hydrofluoric, trifluoroacetic, sulfuric, phosphoric, acetic, succinic, citric, lactic, maleic, fumaric, palmitic, cholic, pamoic, mucic, D-glutamic, D-camphoric, glutaric, phthalic, tartaric, lauric, stearic, salicylic, methanesulfonic, benzenesulfonic, sorbic, picric, benzoic, cinnamic, and like acids.

The present invention also includes the use of solvates of Compound I and salts thereof. "Solvate" means a physical association of a compound of the invention with one or more solvent molecules. This physical association involves varying degrees of ionic and covalent bonding, including hydrogen bonding. In certain instances, the solvate will be capable of isolation. "Solvate" encompasses both solution-phase and isolatable solvates.

### Therapeutic Use

In one aspect, the present invention provides the pharmaceutical composition as described above for use in treating or preventing cancer, a pre-cancerous condition or a benign hyperproliferative disorder.

In some embodiments, the compound of Formula I is useful for treating psoriasis.

In some embodiments, the compound of Formula I is useful for treating actinic keratosis.

In other embodiments, the cancer is skin cancer selected from melanoma and non-melanoma skin cancer.

In other embodiments, the non-melanoma skin cancer is selected from basal cell carcinoma (BCC) and squamous cell carcinoma (SCC).

In some embodiments, the composition can be administered in combination with at least one other active agent.

In another embodiment, the topical pharmaceutical composition comprising Compound I as described herein may be administered with a non-topical pharmaceutical composition comprising Compound I. In one embodiment, the non-topical pharmaceutical composition comprises a composition formulated for intravenous or subcutaneous administration. The non-topical pharmaceutical compositions can be, in another embodiment, administered to a subject by any method known to a person skilled in the art, such as parenteral, intramuscular, intra-dermal, intra-nasal, oral, ophthalmic, or intra-peritoneal administration. In one embodiment, the topical and non-topical pharmaceutical compositions comprising Compound I are administered simultaneously. In another embodiment, the topical and non-topical pharmaceutical compositions comprising Compound I are administered sequentially. In one embodiment, the topical formulation is administered prior to the administration of the non-topical formulation. In another embodiment, the non-topical formulation is administered prior to the administration of the topical formulation.

In some embodiments, the composition is administered one time per day. In other embodiments, the composition is administered 2, 3, or 4 times per day. For example, in some embodiments, the composition is administered 2 times per day. In some embodiments, the composition is administered one time per day. In other embodiments, the composition is administered 3 times per day. In other embodiments, the composition is administered 4 times per day.

In some embodiments, the composition as described herein is administered once every 2 days, once every 3 days, once every 4 days, once every 5 days, once every 6 days, once every 7 days, once every 8 days, once every 9 days, or once every 10 days.

In some embodiments, the composition as described herein is administered once every 2 days. In some embodiments, the composition is administered once every 3 days. In some embodiments, the composition is administered once every 4 days. In some embodiments, the composition is administered once every 5 days. In some embodiments, the composition is administered once every 6 days. In some embodiments, the composition is administered once every 7 days. In some embodiments, the composition is administered once every 8 days. In some embodiments, the composition is administered once every 9 days. In some embodiments, the composition is administered once every 10 days.

In some embodiments, the composition as described herein is administered once every week, once every 2 weeks, once every 3 weeks, once every 4 weeks, once every 5 weeks, once every 6 weeks, once every 7 weeks, or once every 8 weeks. In some embodiments, the composition is administered once every week. In some embodiments, the composition is administered once every 2 weeks. In some embodiments, the composition is administered once every other week. In some embodiments, the composition is administered once every 3 weeks. In some embodiments, the composition is administered once every 4 weeks. In some embodiments, the composition is administered once every 5 weeks. In some embodiments, the composition is administered once every 6 weeks. In some embodiments, the composition is administered once every 7 weeks. In some embodiments, the composition is administered once every 8 weeks.

In some embodiments, the composition is administered once every month. In some embodiments, the composition is administered once every 2 months. In some embodiments, the composition is administered once every other month. In some embodiments, the composition is administered once every 3 months. In some embodiments, the composition is administered once every 4 months. In some embodiments, the composition is administered once every 5 months. In some embodiments, the composition is administered once every 6 months. In some embodiments, the composition is administered once every 7 months. In some embodiments, the composition is administered once every 8 months. In some embodiments, the composition is administered once every 9 months. In some embodiments, the composition is administered once every 10 months. In some embodiments, the composition is administered once every 11 months. In some embodiments, the composition is administered once every 12 months.

In some embodiments, the composition as described herein is administered on a schedule as described hereinabove (e.g., daily, weekly, monthly) with planned or unplanned hiatuses in the regimen. According to this aspect and in one embodiment, the composition is administered for one week and then not administered for one week. In another embodiment, the composition is administered for one week and then not administered for two weeks. In another embodiment, the composition is administered for one week and then not administered for three weeks.

In another embodiment, the composition is administered for two weeks and then not administered for one week. In another embodiment, the composition is administered for two weeks and then not administered for two weeks. In another embodiment, the composition is administered for two weeks and then not administered for four weeks.

In another embodiment, the composition is administered for one month and then not administered for one month. In another embodiment, the composition is administered for one month and then not administered for two months. In another embodiment, the composition is administered for one month and then not administered for three months.

In another embodiment, the composition is administered for two months and then not administered for one month. In another embodiment, the composition is administered for two months and then not administered for two months. In another embodiment, the composition is administered for two months and then not administered for four months.

In some embodiments, the composition as described herein is administered for a total duration of 1-3 months. In one embodiment, the treatment regimen lasts up to 28 days. In another embodiment, the treatment regimen lasts up to 56 days. In another embodiment, the treatment regimen lasts up to 84 days.

In some embodiments, the composition as described herein is administered for a total duration of up to 1 week. In another embodiment, the compositions are administered for up to 2 weeks. In another embodiment, the compositions are administered for up to 3 weeks. In another embodiment, the compositions are administered for up to 1 month. In another embodiment, the compositions are administered for up to 2 months. In another embodiment, the compositions are administered for up to 3 months. In another embodiment, the compositions are administered for up to 4 months. In another embodiment, the compositions are administered for up to 5 months. In another embodiment, the compositions are administered for up to 6 months. In another embodiment, the compositions are administered for up to 9 months. In another embodiment, the compositions are administered for up to 1 year. In another embodiment, the compositions are administered for up to 18 months. In another embodiment, the compositions are administered for up to 2 years.

In one embodiment, the pharmaceutical compositions comprise an effective amount of Compound I. The term "therapeutically effective amount" or "an effective amount" as used herein refers, in one embodiment, to a quantity of a compound which is sufficient to provide a beneficial effect to the subject to which the compound is administered. The effective amount, according to the principles of the present invention can be determined by any one of ordinary skill in the art and can be tested on various models both *in vitro* and *in vivo.*

The term "pharmaceutically acceptable" means in one embodiment, that the compound is approved by a regulatory agency of the Federal or a state government or listed in the U. S. Pharmacopeia or other generally recognized pharmacopeia for use in animals and, more particularly, in humans. Pharmaceutically acceptable non-aqueous excipient in the context of the present invention refers to an approved ingredient which is not water or water-based material.

The term "inhibition of proliferation" in relation to cancer cells, in the context of the present invention and in one embodiment, refers to a decrease in at least one of the following: number of cells (due to cell death which may be necrotic, apoptotic or any other type of cell death or combinations thereof) as compared to control; decrease in growth rates of cells, i.e. the total number of cells may increase but at a lower level or at a lower rate than the increase in control; decrease in the invasiveness of cells (as determined for example by soft agar assay) as compared to control even if their total number has not changed; progression from a less differentiated cell type to a more differentiated cell type; a deceleration in the neoplastic transformation; or alternatively the slowing of the progression of the cancer cells from one stage to the next. In one embodiment, the compositions of the present invention prevent, inhibit or suppress formation of metastases. In another embodiment, the compositions of the present invention prevent, inhibit or suppress the spread of metastases. In one embodiment, the compositions of the present invention prevent, inhibit or suppress the spread of metastases to other organs. In one embodiment, the compositions of the present invention prevent, inhibit or suppress the spread of metastases to lymph nodes. In another embodiment, the compositions of the present invention prevent, inhibit or suppress the spread of metastases below the epidermis, which in one embodiment is a dermis cell and, in another embodiment, is a subcutis cell.

As used herein, the term "administering" refers, in one embodiment, to bringing something into contact with a compound of the present invention. In one embodiment, administration may be to cells or tissue cultures, or to living organisms, for example humans. Thus, in one embodiment, the compounds of the present invention are administered to a human subject. In another embodiment, the compounds of the present invention are administered to a mammalian subject, which in one embodiment, is murine, canine, feline, bovine, ovine, or porcine.

As used herein, the term "subject" refers, in one embodiment, to a human subject. In another embodiment, the subject is murine, which in one embodiment, is a mouse, and, in another embodiment, is a rat. In another embodiment, the subject is canine, feline, bovine, ovine, or porcine. In another embodiment, the subject is a mammal, including primates, such as monkeys and humans, horses, cows, sheep, pigs, goats, cats, dogs, rabbits, birds such as turkey, chickens, and ducks, and rodents such as rats, mice, guinea pigs, and hamsters. In a particular embodiment, the mammal to be treated is human. Other subjects include species that are commonly used in scientific research, animal husbandry or as human companions.

The term "treating" as used herein refers, in one embodiment, to alleviation of the adverse effects of the disease or disorder, which alleviation may be manifested by a decrease in at least one of the following: reduction in the number of abnormal epidermal cells (due to cell death which may be necrotic, apoptotic or any other type of cell death or combinations thereof) as compared to control; decrease in proliferation of cells, i.e. the total number of cells may increase but at a lower level or at a lower rate than the increase in control; or decrease in the invasiveness of cells (as determined for example by soft agar assay) as compared to control even if their total number has not changed.

In one embodiment, "treating" as used herein refers to therapeutic treatment. In one embodiment, "preventing", "suppressing" or "inhibiting" as used herein refers to prophylactic or preventative measures, wherein the object is to prevent or lessen the targeted condition or disorder as described herein. Thus, in one embodiment, "treating" refers *inter alia* to delaying progression, expediting remission, inducing remission, augmenting remission, speeding recovery, increasing efficacy of or decreasing resistance to alternative therapeutics or other formulations of the same drug, in one embodiment, given via another route of administration, or a combination thereof. In one embodiment, "preventing" refers, inter alia, to delaying the onset of symptoms, preventing relapse to a disease, decreasing the number or frequency of relapse episodes, increasing latency between symptomatic episodes, or a combination thereof. In one embodiment, "suppressing" or "inhibiting", refers inter alia to reducing the severity of symptoms, reducing the severity of an acute episode, reducing the number of symptoms, reducing the incidence of disease-related symptoms, reducing the latency of symptoms, ameliorating symptoms, reducing secondary symptoms, reducing secondary infections, prolonging patient survival, or a combination thereof.

The term "topical" or "topical formulation" in the context of the present invention comprises a formulation that is applied directly to a particular site on or in the body. In one embodiment, topical administration comprises administration to the skin. In another embodiment, topical administration comprises administration to a mucous membrane (transmucosal). In another embodiment, topical administration comprises administration by inhalation. In another embodiment, topical administration comprises administration to conjunctiva, ear, or tooth.

In another embodiment, the pharmaceutical compositions are administered topically to body surfaces and are thus formulated in a form suitable for topical administration. In some embodiments, the composition as described herein is in the form of an ointment, cream or gel. In certain embodiments, the composition is administered in the form of an ointment. In another embodiment, lotions, drops and the like are formulated for topical administration. In another embodiment, the pharmaceutical composition is administered as a suppository, for example a rectal suppository or a urethral suppository. In another embodiment, the pharmaceutical composition is delivered in a vesicle, e.g. a liposome.

In another embodiment, the pharmaceutical compositions provided herein are controlled-release compositions, i.e., compositions in which the antigen is released over a period of time after administration. Controlled- or sustained-release compositions include formulation in lipophilic depots (e.g. fatty acids, waxes, oils). In another embodiment, the composition is an immediate-release composition, i.e., a composition in which the pharmaceutical composition is released immediately after administration.

In another embodiment, the pharmaceutical composition is delivered in a controlled release system. In another embodiment, the pharmaceutical composition is administered using a transdermal patch, liposomes, or other modes of administration. In another embodiment, polymeric materials are used; e.g. in microspheres in or an implant.

The term "cancer" in the context of the present invention, in one embodiment, includes all types of neoplasm whether in the form of solid or non-solid tumors, and includes both malignant and premalignant conditions as well as their metastasis. In one embodiment, the cancer is any cancer that may be treated with a composition as described herein. In another embodiment, the cancer is any cancer that may be treated with a topical formulation. In one embodiment, the cancer is skin cancer. In another embodiment, the cancer is breast cancer, bladder cancer, which, in one embodiment comprises non-muscle invasive bladder cancer (NMIBC), cervical cancer, anal cancer, or oral cancer, which, in one embodiment comprises oral cavity or oropharyngeal cancer.

The term "treatment of cancer" in the context of the present invention includes, in one embodiment, at least one of the following: a decrease in the rate of growth of the cancer (i.e. the cancer still grows but at a slower rate); cessation of growth of the cancerous growth, i.e., stasis of the tumor growth, and, in one embodiment, the tumor diminishes or is reduced in size. The term also includes reduction in the number of metastasis, reduction in the number of new metastasis formed, slowing of the progression of cancer from one stage to the other and a decrease in the angiogenesis induced by the cancer. In one embodiment, the tumor is totally eliminated.

The term "skin cancer" in the context of the present invention includes, in one embodiment, melanoma and non-melanoma skin cancer. Melanoma refers, in one embodiment, to cancerous growths originate in the pigment-producing melanocytes in the basal layer of the epidermis. Non-melanoma skin cancer includes, in one embodiment, Basal Cell Carcinoma (BCC) which are abnormal, uncontrolled growths or lesions that arise in the skin's basal cells, lining the deepest layer of the epidermis (the outermost layer of the skin) and, in another embodiment, Squamous Cell Carcinoma (SCC), characterized by an uncontrolled growth of abnormal cells arising in the squamous cells, which compose most of the skin's upper layers (the epidermis). In another embodiment, the non-melanoma skin cancer comprises: angiosarcoma, cutaneous T-cell lymphoma (CTCL), dermatofibrosarcoma protuberans, Merkel cell carcinoma, and sebaceous gland carcinoma.

In another embodiment, "cancer" comprises BCC or SCC in organs other than the skin. In one embodiment, the non-skin organ comprises anus, cervix, head and neck, vagina, breast, bladder, esophagus, urinary, prostate, and lung.

The term "pre-cancer" or "pre-malignant" as used herein interchangeably refers, in one embodiment, to diseases, syndromes or other conditions associated with an increased risk of cancer. Pre-cancer conditions in the context of the present invention include, but are not limited to: breast calcifications, vaginal intra-epithelial neoplasia, Barrett's esophagus, atrophic gastritis, dyskeratosis congenital, sideropenic dysphagia, lichen planus, oral submucous fibrosis, solar elastosis, cervical dysplasia, leukoplakia and erythroplakia. In another embodiment, the pre-cancer condition is Bowen's Disease. In another embodiment, the pre-cancer condition is squamous cell carcinoma *in situ.* In another embodiment, the pre-cancer condition is actinic keratosis.

The term "benign hyperproliferative disorder" as used herein refers, in one embodiment, to a condition in which there is an abnormal growth and differentiation of cells and an increase in the amount of organic tissue that results from cell proliferation. The benign hyperproliferative disorder may be attributed to lack of response or inappropriate response to regulating factors, or alternatively to dysfunctional regulating factors. Non-limiting example of benign hyperproliferative disorder are psoriasis, Lentigines (liver spots), and Nevus (mole).

The term "treating benign hyperproliferative disorders" in the context of the present invention includes, in one embodiment, at least one of the following: a decrease in the rate of growth of the lesions; or cessation of growth of the lesions characteristic of the disorder. In one embodiment, the lesions which are characteristic of the hyperproliferative disorder are diminished, reduced in size or totally eliminated. In one embodiment, the pharmaceutical composition comprising Compound I, according to the principles of the present invention, is for use in the treatment of actinic keratosis. Actinic keratosis typically is a sharply outlined verrucous or keratotic growth which may become malignant.

The term "actinic keratoses" as used in the context of the present invention includes, in one embodiment, skin lesions of keratinocytes which are areas of skin in which tissue shows the tendency to develop into cancer, although the tissue in its present state is not cancerous. Epithelial lesions include actinic keratosis (also called solar keratosis or senile keratosis), hypertrophic actinic keratosis, Bowenoid actinic keratosis, arsenical keratosis, hydrocarbon keratosis, thermal keratosis, radiation keratosis, chronic scar keratosis, viral keratosis, actinic cheilitis, Bowen's disease, erythroplaquia of queyrat, oral erythroplaquia, leukoplakia, and intraepidermal epithelialoma.

The term "psoriasis" as used herein refers, in one embodiment, to a condition in which cells build up rapidly on the surface of the skin, forming thick, silvery scales and itchy, dry, red patches or plaques that are sometimes painful. The patches may vary in severity from small and localized to complete body coverage. Injury to the skin can trigger psoriatic skin changes at that spot, which is known as the Koebner phenomenon. In one embodiment, psoriasis is an autoimmune condition. In one embodiment, psoriasis comprises plaque psoriasis, guttate psoriasis, inverse psoriasis, pustular psoriasis, and erythrodermic psoriasis.

The term "treating psoriasis" in the context of the present invention includes, in one embodiment, decreased plaque area, decreased skin cell proliferation, decreased scaliness, decreased pruritus (itchiness), or decreased pain. In one embodiment, the plaques or patches which are characteristic of psoriasis are diminished, reduced in size or totally eliminated. In one embodiment, the pharmaceutical composition comprising Compound I, according to the principles of the present invention, is for use in the treatment of psoriasis. In another embodiment, the pharmaceutical composition comprising Compound I is for use in the prevention, inhibition or suppression of psoriasis, which in one embodiment comprises decreased frequency or severity of flare-ups or increased remission periods.

In some embodiments, the term "Formula I ointment" refers to the composition of the invention. In other embodiments, the term "Formula I ointment" refers to the composition or the formulation as described in Example 2. In some embodiments, the term "Formula I ointment" includes 5% Formula I, 10% Formula I, 15% Formula I, and 20% Formula I. In some embodiments, the terms "5% Formula I" and "Formula I (5%)" are used interchangeably herein; the terms "10% Formula I" and "Formula I (10%)" are used interchangeably herein; the terms "15% Formula I" and "Formula I (15%)" are used interchangeably herein; and the terms "20% Formula I" and "Formula I (20%)" are used interchangeably herein.

### List of Abbreviations

- AE: Adverse event
- AK: Actinic keratosis
- BMI: Body mass index
- CSR: Clinical study report
- IEA: Interim Efficacy Analysis
- IND: Investigational new drug
- LLOQ: Lower limit of quantification
- LSR: Local Skin Reaction
- PK: Pharmacokinetics
- SAE: Serious adverse event
- TEAE: Treatment emergent adverse event

The invention will be further illustrated with reference to the following illustrative examples.

### EXAMPLES

### EXAMPLE 1: Accelerated single combination stability study

Topical formulation of Compound I was carefully designed while considering the chemical sensitivity of Compound I. The stability of Compound I was studied in the presence of inactive ingredients suitable for topical formulation, while water and alcohols which may serve as potential nucleophiles were excluded. Ingredients suitable for being carriers, penetration enhancers, or thickening agents were pre-selected and tested in combination with Compound I: Labrafac PG (propylene glycol dicaprylate); Labrafac lipophile WL1349 (Caprylic/capric triglyceride); Gelucire 43/01 (glyceride and PEG esters); (+)-Limonene; PEG 400 Dioleate; PEG 400 Dilaurate; Oleic acid and DMSO (dimethyl sulfoxide).

A short accelerated single combination (Compound I + 1 excipient) stability study was performed in 40°C and 65% relative humidity (RH).

The amount of Compound I was measured at the following time points: time zero, three days, one week and two weeks, in order to evaluate the compatibility of Compound I with the pre-selected ingredients (Table 1).

**Table 1: Compatibility of Compound I with various excipients**

| **Excipients Name** | **Compound I % of declared amount** | | | |
|---|---|---|---|---|
| | **Time zero** | **3 days** | **1 week** | **2 weeks** |
| Labrafac PG (Propylene glycol dicaprylate) | 97.2 | 97.5 | 96.8 | **92.3** |
| Labrafac Lipophile WL 1349 (Caprylic/capric triglyceride) | 97.3 | 97.2 | 97.6 | **94.1** |
| Gelucire 43/01 | 89.1 | 80.3 | 85.7 | 76.0 |
| R-(+)-Limonene | 98.3 | 92.7 | 82.8 | 72.9 |
| PEG 400 Dioleate (DO) | 96.4 | 93.5 | 90.1 | 81.1 |
| PEG 400 Dilaurate (DL) | 96.9 | 94.4 | 89.5 | 80.3 |
| Oleic acid | 95.0 | 48.9 | 31.4 | 19.6 |
| DMSO | 97.6 | 96.9 | 97.1 | **96.5** |

Results: As shown in Table 1, has been surprisingly found that propylene glycol dicaprylate and caprylic/capric triglyceride are compatible with Compound I in topical formulations, while Compound I was not sufficiently stable in the presence of glyceride/PEG ester mixtures (Gelucire 43/01), PEG esters and the hydrophobic hydrocarbon Limonene. Furthermore, the presence of oleic acid resulted in a sharp degradation of Compound I.

Labrafac PG, Labrafac lipophile WL1349 and DMSO were selected out of this group with the anticipated choice of +5°C as the normal condition storage temperature for the drug product.

### EXAMPLE 2: Topical composition

The three inactive ingredients that were found to stabilize active Compound I are liquids at room temperature, thus white Petrolatum and paraffin wax were introduced in order to get the consistency of a semi-solid ointment, giving rise to the following stable ointment formulation of Compound I:

**Table 2: Composition of 5%, 10%, 15%, and 20% Compound I ointment**

| **Commercial name** | **Equivalent IID name** | **% Used (w/w)** | ***IID limit for topical % (w/w)** |
|---|---|---|---|
| API: Compound I | N/A | 5, 10, 15, 20 | N/A |
| Labrafac PG | Propylene glycol dicaprylate | 10, 7.5, 5, 2.5 | 10 |
| Labrafac lipophile WL 1349 | Caprylic/capric triglyceride | 50, 47.5, 45, 42.5 | 50 |
| DMSO | Dimethyl sulfoxide | 1 | 45 |
| White petrolatum | Petrolatum | 30 | 99.98 |
| Paraffin wax | Paraffin white soft | 4 | 15 |
| SUM | | 100 | |

| | | | |
|---|---|---|---|
| *FDA's database on Inactive Ingredients (IID) | | | |

Inactive ingredients for the ointment formulation of Compound I of the present invention were selected from the FDA's database on Inactive Ingredients (IID) used in approved topical (dermal) use.

### EXAMPLE 3: Phase 1 Study (STUDY A)

### STUDY A: Phase 1 Dose-Escalation Study

### Title of Study

A Phase 1 randomized, double-blinded, placebo-controlled, dose-escalation study in healthy older-adult volunteers to evaluate the safety, tolerability, and pharmacokinetics of a single topical dermal application of Formula I ointment.

### Purpose of Study

Safety, tolerability and pharmacokinetics (PK) of Formula I ointment.

### Study Objectives

To evaluate the systemic and local tolerability and safety of a single topical skin application of Formula I ointment in healthy older-adult volunteers.

To determine the plasma PK profile of the main metabolite of Formula I following a single topical skin application of Formula I ointment in healthy older=adult volunteers.

### Subject Population(s) Under Study

Healthy volunteers, 35 to 70 years of age at the time of screening, with Fitzpatrick skin types I, II, or III, who had at least one 25 cm² area of healthy skin on the forehead (pre-defined criteria).

### Treatment Regimen

A 25 cm² on the forehead was selected as the treatment field.

Subjects were sequentially assigned to 1 of 3 consecutive treatment cohorts (5%, 10%, 15%, or 20% Formula I, respectively). Five subjects were randomized in a double-blind fashion in each dose cohort with 4 subjects to receive active Formula I ointment and 1 subject to receive matched placebo (0% Formula I; vehicle control). Thus, a total of 12 subjects were allocated to receive active Formula I ointment and 3 subjects were allocated to receive placebo. The clinical trial included 3 study periods: Screening, Treatment, and Observation Periods.

### Subject Enrollment Status

### 1. Cumulative Enrollment Summary

**Table 3: Cumulative Enrollment Summary in STUDY A**

| **Enrollment Parameter** | **Total** |
|---|---|
| Total planned accrual: | 15 |
| Number of subjects enrolled: | 15 |
| Number of subjects completed: | 15 |
| Number of subjects that withdrew/withdrawn from study: | 0 |

### 2. Total Number of Enrolled Subjects by Age, Gender, and Race

**Table 4: Subject Listing by Age, Gender, and Race in STUDY A**

| **Subject ID** | **Dose group** | **Date of informed consent** | **Sex** | **Race** | **Age, years** |
|---|---|---|---|---|---|
| 10101 | Formula I (5%) | 09Mar2016 | M | Caucasian | 48.7 |
| 10102 | Formula I (5%) | 10 Mar2016 | M | Caucasian | 46.8 |
| 10103 | Formula I (5%) | 10 Mar2016 | M | Caucasian | 57.3 |
| 10104 | Formula I (5%) | 11 Mar2016 | M | Caucasian | 56.8 |
| 10105 | Placebo | 09 Mar2016 | F | Caucasian | 65.7 |
| 10106 | Formula I (10%) | 18 Mar2016 | M | Caucasian | 42.9 |
| 10107 | Placebo | 21 Mar2016 | M | Caucasian | 41.2 |
| 10108 | Formula I (10%) | 01Apr2016 | F | Caucasian | 69.4 |
| 10109 | Formula I (10%) | 31 Mar2016 | M | Caucasian | 65.6 |
| 10110 | Formula I (10%) | 05Apr2016 | M | Caucasian | 35.6 |
| 10111 | Formula I (20%) | 11Apr2016 | M | Caucasian | 43.1 |
| 10112 | Formula I (20%) | 18Apr2016 | M | Caucasian | 56.7 |
| 10113 | Formula I (20%) | 19Apr2016 | M | Caucasian | 54.3 |
| 10114 | Placebo | 19Apr2016 | F | Caucasian | 49.9 |
| 10115 | Formula I (20%) | 03May2016 | F | Caucasian | 61.9 |

**Table 5: Number of Enrolled Subjects by Age, Gender, and Race in STUDY A**

| **Demographic parameter** | **Category** | **Total (N=15)** |
|---|---|---|
| | | **n (%)** |
| Sex | Female | 4 (26.7) |
| | Male | 11 (73.3) |
| Age | <35 years | 0 |
| | 35-40 years | 1 (6.7) |
| | 41-50 years | 6 (40.0) |
| | 51-60 years | 4 (26.7) |
| | 61-70 years | 4 (26.7) |
| Female by age group (n=4) | <35 years | 0 |
| | 35-40 years | 0 |
| | 41-50 years | 1 (25.0) |
| | 51-60 years | 0 |
| | 61-70 years | 3 (75.0) |
| Male by age group (n=11) | <35 years | 0 |
| | 35-40 years | 1 (9.1) |
| | 41-50 years | 5 (45.5) |
| | 51-60 years | 4 (36.4) |
| | 61-70 years | 1 (9.1) |
| Race | Caucasian | 15 (100) |

### STUDY A Results

### Demographics and Disposition

A total of 15 subjects were enrolled in this study (12 active and 3 placebo subjects) and no subject discontinued prematurely. Generally, treatment groups were similar in terms of demographic characteristics except for gender. Two of 3 placebo treated subjects were female; no female subject received Formula I (5%); 1 female subject received Formula I (10%) and 1 female subject received Formula I (20%). All subjects were Caucasian. Subject ages ranged between 35.6 and 69.4 years. The mean age of the Formula I ointment-treated subjects was 53.3 years and the mean age of the pooled placebo-treated subjects was 52.3 years. The mean BMI value for all Formula I ointment treated subjects was 30.1 kg/m² (range between 24.5 and 34.3 kg/m²) and for the pooled placebo-treated subjects was 28.5 kg/m² (range between 22.9 and 33.1 kg/m²).

### Pharmacokinetics

Plasma concentrations of Formula I (LLOQ of 1 ng/mL) and jasmonic acid (LLOQ of 50 ng/mL) at all time points were below the LLOQ. Thus, no PK analyses were performed.

### Safety

*Exposure and Dosing:* Each subject received their assigned treatment. Subjects in the Formula I (5%) cohort were inadvertently under-dosed (mean amount of study drug was 195 mg), while the mean amount for the Formula I 10% and 20% cohorts was 254 mg and 247 mg, respectively (target amount was 250 mg).

*Adverse Events (AEs):* There were no deaths, serious adverse events (SAEs), or early study discontinuations due to AEs in this study. There were a total of 6 treatment-emergent AEs (TEAEs) reported. All the AEs were mild in severity. There were no dose-related or temporally-related patterns of AEs.

The only AE deemed related to the study drug by the investigator was in a single subject in Cohort 2 (Formula I (10%)). The subject reported mild "left eye pain" approximately 4 hours after the study drug was applied to the forehead (subject received Formula I (10%)). This event was short lived and resolved without sequelae.

*Clinical Laboratory Evaluation:* There were no clinically significant laboratory abnormalities or changes in laboratory parameters for any subject at any time point.

*Vital Signs, Physical Findings, and Other Observations Related to Safety:* There were no clinically significant changes in vital signs, physical examinations, or ECGs for any individual subject at any time point regardless of treatment.

Examinations and rating of the Treatment Field for each subject during the 24-hour treatment period and for up to 7 days after the study drug application were performed for signs of erythema, edema, weeping/exudate, vesicles, erosion/ulceration, flaking/scaling/dryness, scabbing/crusting, itching, or pain. There were no treatment emergent findings for any subject for any assessment with the exception of mild flaking/scaling/dryness on Day 3 for a single subject receiving Formula I (20%) and mild pain on Day 7 for a single subject receiving placebo.

### Progress Information

**STUDY A** (conducted in healthy subjects) has been completed and the treatment code has been unblinded.

### Most Frequent Adverse Events

TEAEs were reported for 4 (33.3%) subjects in any Formula I ointment group and 2 (66.7%) subjects in the placebo group (Table 6). No SAE was reported during the study. The frequencies of TEAEs by system organ class and preferred term are summarized in Table 7.

**Table 6: Summary of Adverse Event Frequency by Treatment in STUDY A**

| **Category** | **Treatment** | | | |
|---|---|---|---|---|
| | **Pooled Placebo (N = 3)** | **Formula I (5%) (N = 4)** | **Formula I (10%) (N = 4)** | **Formula I (20%) (N = 4)** |
| **n (%)** | | | | |
| Any AE | 2 (67%) | 2 (50%) | 2 (50%) | 0 |
| Any TEAE | 2 (67%) | 2 (50%) | 1 (25%) | 0 |
| Any TEAE related to treatment | 0 | 0 | 1 (25%) | 0 |
| Any SAE | 0 | 0 | 0 | 0 |

**Table 7: Summary of the Frequency of TEAEs by System Organ Class and Preferred Term by Treatment in STUDY A**

| **System Organ Class Preferred Term** | **Treatment** | | | |
|---|---|---|---|---|
| | **Pooled Placebo (N = 3)** | **Formula I (5%) (N = 4)** | **Formula (10%) (N = 4)** | **Formula I (20%) (N = 4)** |
| **n (%)** | | | | |
| Any TEAE | 2 (67%) | 2 (50%) | 1 (25%) | 0 |
| Eye disorders | 0 | 1 (25%) | 1 (25%) | 0 |
| Eye irritation | 0 | 1 (25%) | | 0 |
| Eye pain | 0 | 0 | 1 (25%) | 0 |
| Gastrointestinal disorders | 1 (33%) | 0 | 0 | 0 |
| Nausea | 1 (33%) | 0 | 0 | 0 |
| Musculoskeletal and connective tissue disorders | 1 (33%) | 0 | 0 | 0 |
| Back pain | 1 (33%) | 0 | 0 | 0 |
| Skin and subcutaneous tissue disorders | 1 (33%) | 1 (25%) | 0 | 0 |
| Skin abrasion | 1 (33%) | 1 (25%) | 0 | 0 |

### Deaths and Serious Adverse Events besides Death

There were no deaths or SAEs during study **STUDY A.**

### Study Subject Dropouts Due to an Adverse Drug Experience

There were no early discontinuations due to AEs in STUDY A.

### Summary of Information Obtained Pertinent to Understanding the Drug's Actions (Dose Response, Bioavailability, Information from Other Controlled Trials, etc.)

Twelve subjects underwent timed blood samplings for PK analysis in **STUDY A**. All samples collected were below the LLOQ for Formula I (1 ng/mL) and its major metabolite jasmonic acid (50 ng/mL).

In **STUDY A**, there were no safety related findings that suggested a dose response pattern at the Formula I doses evaluated versus placebo.

### EXAMPLE 4: Phase 2 Study in subjects with actinic keratosis

### Title of Study

A Phase 2 randomized, double-blind, placebo-controlled, parallel-cohort study to evaluate the efficacy, safety, tolerability, and pharmacokinetics of once-daily application of topical Formula I ointment for 28 days in subjects with actinic keratosis.

### Purpose of Study

Safety and efficacy.

### Study Objectives

To compare the reduction on Day 56 in the number of the actinic keratosis (AK) lesions in the Treatment Field of subjects receiving once-daily topical 5% or 10% Formula I ointment for 28 days to the reduction in the number of AK lesions in subjects receiving placebo.

To evaluate the systemic and local (skin) safety and tolerability of once-daily topical application of 5% or 10% Formula I ointment or placebo for 28 days in adult subjects with AK.

To assess the systemic exposure of Formula I and jasmonic acid, its primary metabolite, at selected time points during topical application of 5% or 10% Formula I ointment or placebo for 28 days in adult subjects with AK.

To compare the proportion of subjects having complete clearance of AK lesions on Day 56 between subjects receiving 28 days of 5% or 10% Formula I ointment and subjects receiving placebo.

To compare the reduction on Day 84 in the number of the actinic keratosis (AK) lesions in the Treatment Field of subjects receiving once-daily topical 5% or 10% Formula I ointment for 28 days to the reduction in the number of AK lesions in subjects receiving placebo.

### Subject Population(s) Under Study

Subjects, 18 years and older, who at baseline (Screening and Day 1 pre-dose visits) had a minimum of 4 and a maximum of 8 discrete Grade 1-2 AK lesions within a single 25 cm² area of skin on their scalp or face.

### Treatment Regimen

The Treatment Field is selected at screening and confirmed on Day 1 pre-dose. Any 25 cm² contiguous area on the face or scalp of the subject that contains 4-8 AK lesions that conform to AK lesion eligibility criteria as the study Treatment Field.

Subjects are randomly assigned in a double-blind fashion to 1 of 3 parallel treatment cohorts (5% Formula I ointment, 10% Formula I ointment, or placebo, respectively) in a ratio of 1:1:1. Randomized subjects apply a single, thin application of the study drug to their treatment field for 28 days.

### Subject Enrollment Status

### 1. Enrollment Time Table

**Table 8: Enrollment Time Table in the Phase 2 study**

| | |
|---|---|
| Date study open to enrollment: | July 28, 2016 |
| Date enrollment closed: | February 02, 2017 |
| Expected end date of study: | April 27, 2017 |

### 2. Cumulative Enrollment Summary

**Table 9: Cumulative Enrollment Summary in the Phase 2 study**

| | **All Subjects^{a}** |
|---|---|
| | **n (%)** |
| Total planned accrual: | 84 (75 evaluable) |
| Number of subjects enrolled to date ^{b}: | 92 (100) |
| Number of subjects completed to date ^{b}: | 60 (65.2) |
| Number of subjects that withdrew/withdrawn from study to date ^{b} | 1 (1.1) |

| | |
|---|---|
| ^{a} Subjects were randomly assigned to 5% or 10% Formula I ointment or placebo. ^{b} Cut-off date of 04 February 2017 One subject withdrew consent on study day 56 due to his extensive winter vacation plans. The subject experienced no AEs during his time enrolled in the study. | |

### 3. Total Number of Enrolled Subjects by Age, Gender, and Race

The study has enrolled 82 (89.1%) male patients and 10 (10.9%) female patients. The majority of patients (58.7%) were ≥65 years of age, and all 92 patients (100%) were Caucasian.

**Table 10: Number of Enrolled Subjects by Age, Gender, and Race in the Phase 2 study**

| **Demographic parameter** | | **All Subjects^{a}** |
|---|---|---|
| | | **n (%)** |
| Sex | Female | 10 (10.9) |
| | Male | 82 (89.1) |
| Age | 18-29 years | 0 |
| | 30-49 years | 2 (2.2) |
| | 50-64 years | 36 (39.1) |
| | 65-74 years | 38 (41.3) |
| | 75-84 years | 14 (15.2) |
| | ≥ 65 years | 54 (58.7) |
| | ≥75 years | 16 (17.4) |
| | ≥ 85 years | 2 (2.2) |
| Female by age group | 18-29 years | 0 |
| | 30-49 years | 0 |
| | 50-64 years | 5 (50) |
| | 65-74 years | 3 (30) |
| | 75-84 years | 2 (20) |
| | ≥65 years | 5 (50) |
| | ≥75 years | 2 (20) |
| | ≥85 years | 0 |
| Male by age group | 18-29 years | 0 |
| | 30-49 years | 2 (2.4) |
| | 50-64 years | 31 (37.8) |
| | 65-74 years | 35 (42.7) |
| | 75-84 years | 12 (14.6) |
| | ≥65 years | 49 (59.8) |
| | ≥75 years | 14 (17.1) |
| | ≥85 years | 2 (2.4) |
| Race | Caucasian | 92 (100) |
| | Black or African American | 0 |
| | Asian | 0 |
| | Native Hawaiian or Other Pacific Islander | 0 |
| | American Indian or Alaska Native | 0 |
| | Other | 0 |
| | Race Not Reported | 0 |
| Ethnicity | Hispanic or Latino | 0 |
| | Not Hispanic or Latino | 92 (100) |

| | | |
|---|---|---|
| ^{a} Subjects were randomly assigned to 5% or 10% Formula I ointment or placebo. | | |

### Phase 2 Study Results

The Phase 2 study is currently ongoing; therefore, the study is still blinded. There have been no deaths or reportable SAEs during the reporting period.

The first 15 (PK and non-PK) subjects enrolled in the trial underwent the safety examinations planned for all other subjects on Day 7 plus physical examination, clinical laboratory and ECG assessments. Once all 15 subjects in this Nested Phase 1b Safety Sub-Cohort completed their Day 7 Visit, safety data (including AEs, Local Skin Reaction (LSR) scores, clinical laboratory results, and ECG data) from Day 1 through Day 7 were reviewed in a blinded fashion by a safety committee consisting of 3 Board-certified internists and sub-specialists. No AEs had been recorded and no safety signal was found. Local skin reactions were unremarkable with no individual LSR above mild.

A single interim efficacy analysis (IEA) was conducted as soon as approximately 50% of the subjects had completed their Day 56 Visit. The objective of the IEA is to evaluate the probability of achieving the primary efficacy endpoint at the conclusion of the study. The unblinded data for the interim analysis sub-set was analyzed by an independent statistician and found that, based upon the data available at the time of the IEA, there was a chance for at least one of the two treatment arms to achieve the primary endpoint.

### Progress Information

The Phase 2 study is being conducted in a target patient population; this study is ongoing, and the treatment code is still blinded.

### Most Frequent and Most Serious Adverse Events

**Table 11: Serious Adverse Events in the Phase 2 study**

| **MedDRA System Organ Class Preferred term** | **All subjects^{a}** |
|---|---|
| | **n(%)** |
| Number of subjects enrolled | 92 (100) |
| Any serious adverse event | 2 (2.2) |
| Infections and infestations | 1 (1.1) |
| Pneumonia | 1 (1.1) |
| Neoplasms benign, malignant and unspecified (including cysts and polyps) | 1 (1.1) |
| Benign sero-mucinous cystadenoma | 1 (1.1) |

| | |
|---|---|
| ^{a} Subjects were randomly assigned to 5% or 10% Formula I ointment or placebo. | |

There have been no deaths, suspected unexpected SAEs, study drug related SAEs, or severe AEs reported.

Two SAEs were reported in this trial (Table 11). Neither was deemed related to the study drug by the Investigator or the Sponsor.

The first subject was a 77-year-old female who developed a colo-vaginal fistula in May, 2016 (3 months prior to screening). The subject underwent subsequent laparoscopic sigmoid colectomy on 29 June 2017 at which time a mucinous cystadenoma was incidentally found. The subject scheduled elective out-patient salpingo-oophorectomy for 28 September, but failed to communicate this information to the site personnel at the Screening Visit on 22 August. Subsequently, the subject was enrolled on 30 August and she administered her final study drug application on Day 27 (26 September 2017). At the Day 28 Visit on 27 September, the subject notified the site that she was planning to have elective surgery the next day. Surgery proceeded as planned and was uneventful; however, the subject was hospitalized overnight after the surgeon learned that she would be alone at home overnight. A benign sero-mucinous cystadenoma was reported by the pathologist who examined the specimens removed at surgery. The Investigator and the Sponsor agreed that the SAE was unrelated to the study drug since the surgical condition was diagnosed approximately 2 months prior to randomization.

The second subject was an 81-year-old male with a past medical history of Hemophilia A and asthma who was hospitalized 4 days for treatment of bilateral lower lobe pneumonia during which the study drug was temporarily discontinued. The subject recovered with no sequalae and re-started the study drug upon discharge from the hospital. The Investigator and Sponsor agreed that the SAE was unrelated to the study drug.

### Study Subject Deaths

There have been no deaths reported in the Phase 2 study.

### Study Subject Dropouts Due to an Adverse Drug Experience

One subject discontinued treatment prematurely due to an AE that was judged by the Investigator and Sponsor to be unrelated to the study treatment.

The subject is a 63-year-old white male with a past medical history of coronary heart disease, hypercholesterolemia, diverticulitis, and Fuchs' Corneal Endothelial Dystrophy and whose daily medications were fluorometholone 0.1% eyedrops, aspirin 81 mg orally, and rosuvastatin 10 mg orally. The subject reported mild right eye fatigue on Day 12 and discontinued administration of the study drug on Day 14. The AE ended 2 days later without any intervention or sequelae. Despite discontinuing the study drug, the subject continued his full participation in the trial follow-up and procedures until his scheduled exit. The subject reported 2 additional AEs of constipation and diarrhea on Day 17, three days after discontinuing Formula I ointment, that were mild, self-limited, and were also deemed unrelated to the study drug by the Investigator and Sponsor.

### Summary of Information Obtained Pertinent to Understanding the Drug's Actions (Dose Response, Bioavailability, Information from Other Controlled Trials, etc.)

Twelve subjects underwent timed blood samplings for PK analysis in the Phase 2 study. All samples collected were below the LLOQ for Formula I(1 ng/mL) and its major metabolite jasmonic acid (20 ng/mL).

## Claims

1. A topical pharmaceutical composition comprising a therapeutically effective amount of a compound represented by the structure of Formula I, or a pharmaceutically acceptable salt or solvate thereof, and at least one non-aqueous, pharmaceutically acceptable excipient comprising a glycol diester with a saturated or unsaturated fatty acid, a triglyceride and an aprotic organic solvent

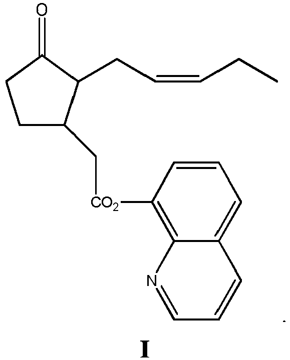

2. The composition according to claim 1, wherein said triglyceride comprises a compound represented by the structure: 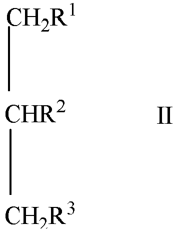 wherein:
R¹, R² and R³ are each R⁴-COO-; and
R⁴-COO- is the residue of a saturated or unsaturated fatty acid.

3. The composition according to any one of claims 1-2, wherein:
said organic solvent is dimethylsulfoxide (DMSO);
said triglyceride is caprylic/capric triglyceride; and/or
said glycol diester with a saturated or unsaturated fatty acid is a propylene glycol diester, such as a propylene glycol dicaprylate.

4. The composition according to claim 3, wherein each of said fatty acids in said propylene glycol diester or in said triglyceride is a C₈-C₁₂ saturated or unsaturated fatty acid.

5. The composition according to any one of claims 1-4, further comprising an oil, a wax, or a combination thereof.

6. The composition according to any one of claims 1-5, comprising a compound of Formula I, propylene glycol dicaprylate, caprylic/capric triglyceride, dimethylsulfoxide (DMSO), petrolatum and paraffin wax.

7. The composition according to any one of claims 1-6, comprising a compound of Formula I in an amount of from about 5% w/w to about 30% w/w.

8. The composition according to claim 7, comprising:
a compound of Formula I in an amount of about 5% to 30% w/w;
propylene glycol dicaprylate in an amount of about 1% to about 10% w/w;
caprylic/capric triglyceride in an amount of about 30% to about 50% w/w;
dimethyl sulfoxide in an amount of about 0% to about 10% w/w;
petrolatum in an amount of about 20% to about 50% w/w; and
paraffin wax in an amount of about 0 to about 10% w/w.

9. The composition according to claim 8, comprising:
a compound of Formula I in an amount of about 5% w/w;
propylene glycol dicaprylate in an amount of about 10% w/w;
caprylic/capric triglyceride in an amount of about 50% w/w;
dimethyl sulfoxide in an amount of about 1% w/w;
petrolatum in an amount of about 30% w/w; and
paraffin wax in an amount of about 4% w/w;
or
a compound of Formula I in an amount of about 10% w/w;
propylene glycol dicaprylate in an amount of about 7.5% w/w;
caprylic/capric triglyceride in an amount of about 47.5% w/w;
dimethyl sulfoxide in an amount of about 1% w/w;
petrolatum in an amount of about 30% w/w; and
paraffin wax in an amount of about 4% w/w;
or
a compound of Formula I in an amount of about 15% w/w;
propylene glycol dicaprylate in an amount of about 5% w/w;
caprylic/capric triglyceride in an amount of about 45% w/w;
dimethyl sulfoxide in an amount of about 1% w/w;
petrolatum in an amount of about 30% w/w; and
paraffin wax in an amount of about 4% w/w;
or
a compound of Formula I in an amount of about 20% w/w;
propylene glycol dicaprylate in an amount of about 2.5% w/w;
caprylic/capric triglyceride in an amount of about 42.5% w/w;
dimethyl sulfoxide in an amount of about 1% w/w;
petrolatum in an amount of about 30% w/w; and
paraffin wax in an amount of about 4% w/w.

10. The composition according to any one of claims 1-9, in the form of an ointment, cream or gel.

11. The composition according to any one of claims 1-10, wherein said composition is essentially free of water, alcoholic solvents and carboxylic acids.

12. The composition according to any one of claims 1-11, for use in treating or preventing cancer, a pre-cancerous condition or a benign hyperproliferative disorder.

13. The composition for use according to claim 12, wherein said condition or disorder is psoriasis or actinic keratosis.

14. The composition for use according to claim 12, wherein said cancer is skin cancer.

15. The composition for use according to claim 14, wherein said skin cancer comprises a melanoma or a non-melanoma skin cancer.

16. The composition for use according to claim 15, wherein said non-melanoma skin cancer comprises basal cell carcinoma (BCC), squamous cell carcinoma (SCC), or cutaneous T-cell lymphoma (CTCL).

## Patentansprüche

1. Topische pharmazeutische Zusammensetzung, umfassend eine therapeutisch wirksame Menge einer durch die Struktur der Formel I dargestellten Verbindung oder ein pharmazeutisch akzeptables Salz oder Solvat davon und mindestens ein nicht wässriges, pharmazeutisch akzeptables Hilfsmittel, umfassend ein Glycoldiester mit einer gesättigten oder ungesättigten Fettsäure, einem Triglycerid und einem aprotischen organischen Lösungsmittel:

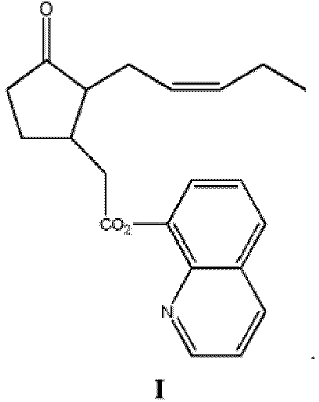

2. Zusammensetzung nach Anspruch 1, wobei das Triglycerid eine durch die folgende Struktur dargestellte Verbindung umfasst: 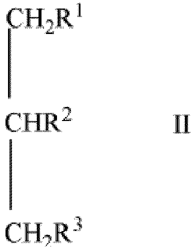 wobei
R¹, R² und R³ jeweils R⁴-COO- ist; und
R⁴-COO- ein Rückstand einer gesättigten oder ungesättigten Fettsäure ist.

3. Zusammensetzung nach einem der Ansprüche 1-2, wobei
das organische Lösungsmittel Dimethylsulfoxid (DMSO) ist;
das Triglycerid Capryl/Caprin-Triglycerid ist; und/oder
das Glycoldiester mit einer gesättigten oder ungesättigten Fettsäure ein PropylenGlycoldiester, wie etwa ein Propylenglycoldicaprylat, ist.

4. Zusammensetzung nach Anspruch 3, wobei jede der Fettsäuren in dem Propylenglycoldiester oder in dem Triglycerid eine C₈-C₁₂ gesättigte oder ungesättigte Fettsäure ist.

5. Zusammensetzung nach einem der Ansprüche 1-4, ferner umfassend ein Öl, ein Wachs oder eine Kombination davon.

6. Zusammensetzung nach einem der Ansprüche 1-5, umfassend eine Verbindung der Formel I, Propylenglycoldicaprylat, Capryl/Caprin-Triglycerid, Dimethylsulfoxid (DMSO), Petrolatum und Paraffinwachs.

7. Zusammensetzung nach einem der Ansprüche 1-6, umfassend eine Verbindung der Formel I in einer Menge von 5 Gew.-% bis 30 Gew.-%.

8. Zusammensetzung nach Anspruch 7, umfassend wie folgt:
eine Verbindung der Formel I in einer Menge von etwa 5 Gew.-% bis etwa 30 Gew.-%;
Propylenglycoldicaprylat in einer Menge von etwa 1 Gew.-% bis etwa 10 Gew.-%;
Capryl/Caprin-Triglycerid in einer Menge von etwa 30 Gew.-% bis etwa 50 Gew-%; Dimethylsulfoxid in einer Menge von etwa 0 Gew.-% bis etwa 10 Gew.-%;
Petrolatum in einer Menge von etwa 20 Gew.-% bis etwa 50 Gew.-%; und
Paraffinwachs in einer Menge von etwa 0 Gew.-% bis etwa 10 Gew.-%.

9. Zusammensetzung nach Anspruch 8, umfassend wie folgt:
eine Verbindung der Formel I in einer Menge von etwa 5 Gew.-%;
Propylenglycoldicaprylat in einer Menge von etwa 10 Gew.-%;
Capryl/Caprin-Triglycerid in einer Menge von etwa 50 Gew.-%;
Dimethylsulfoxid in einer Menge von etwa 1 Gew.-%;
Petrolatum in einer Menge von etwa 30 Gew.-%; und
Paraffinwachs in einer Menge von etwa 4 Gew.-%;
oder
eine Verbindung der Formel I in einer Menge von etwa 10 Gew.-%;
Propylenglycoldicaprylat in einer Menge von etwa 7,5 Gew.-%;
Capryl/Caprin-Triglycerid in einer Menge von etwa 47,5 Gew.-%;
Dimethylsulfoxid in einer Menge von etwa 1 Gew.-%;
Petrolatum in einer Menge von etwa 30 Gew.-%; und
Paraffinwachs in einer Menge von etwa 4 Gew.-%;
oder
eine Verbindung der Formel I in einer Menge von etwa 15 Gew.-%;
Propylenglycoldicaprylat in einer Menge von etwa 5 Gew.-%;
Capryl/Caprin-Triglycerid in einer Menge von etwa 45 Gew.-%;
Dimethylsulfoxid in einer Menge von etwa 1 Gew.-%;
Petrolatum in einer Menge von etwa 30 Gew.-%; und
Paraffinwachs in einer Menge von etwa 4 Gew.-%;
oder
eine Verbindung der Formel I in einer Menge von etwa 20 Gew.-%;
Propylenglycoldicaprylat in einer Menge von etwa 2,5 Gew.-%;
Capryl/Caprin-Triglycerid in einer Menge von etwa 42,5 Gew.-%;
Dimethylsulfoxid in einer Menge von etwa 1 Gew.-%;
Petrolatum in einer Menge von etwa 30 Gew.-%; und
Paraffinwachs in einer Menge von etwa 4 Gew.-%.

10. Zusammensetzung nach einem der Ansprüche 1-9 in der Form einer Salbe, einer Creme oder eines Gels.

11. Zusammensetzung nach einem der Ansprüche 1-10, wobei die Zusammensetzung im Wesentlichen frei von Wasser, alkoholischen Lösungsmitteln und Carbonsäuren ist.

12. Zusammensetzung nach einem der Ansprüche 1-11 zur Anwendung beim Behandeln oder Vorbeugen von Krebserkrankungen, einem präkanzerösen Zustand oder eines gutartigen hyperproliferativen Krankheitsbilds.

13. Zusammensetzung nach Anspruch 12, wobei der Zustand oder das Krankheitsbild Psoriasis oder aktinische Keratose ist.

14. Zusammensetzung nach Anspruch 12, wobei die Krebserkrankung Hautkrebs ist.

15. Zusammensetzung nach Anspruch 14, wobei der Hautkrebs ein Melanom oder einen Hautkrebs, der kein Melanom ist, umfasst.

16. Zusammensetzung nach Anspruch 15, wobei der Hautkrebs, der kein Melanom ist, ein Basaliom (SCC), ein Plattenepithelkarzinom (SCC) oder ein kutanes T-Zell-Lymphom (CTCL) umfasst.

## Revendications

1. Composition pharmaceutique topique comprenant une quantité thérapeutiquement efficace d'un composé représenté par la structure de formule I, ou d'un sel ou solvate pharmaceutiquement acceptable de celui-ci, et au moins un excipient non aqueux, pharmaceutiquement acceptable comprenant un diester de glycol avec un acide gras saturé ou insaturé, un triglycéride et un solvant organique aprotique

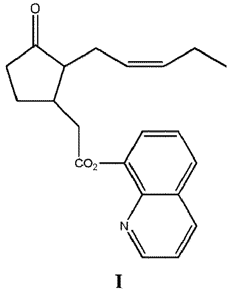

2. Composition selon la revendication 1, dans laquelle ledit triglycéride comprend un composé représenté par la structure : 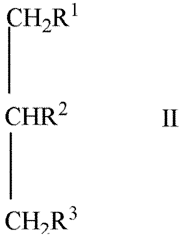 dans lequel :
R¹, R² et R³ sont chacun R⁴-COO- ; et
R⁴-COO- est le résidu d'un acide gras saturé ou insaturé.

3. Composition selon l'une quelconque des revendications 1 à 2, dans laquelle :
ledit solvant organique est le diméthylsulfoxyde (DMSO) ;
ledit triglycéride est un triglycéride caprylique / caprique ; et/ou
ledit diester de glycol avec un acide gras saturé ou insaturé est un diester de propylène glycol, tel qu'un dicaprylate de propylène glycol.

4. Composition selon la revendication 3, dans laquelle chacun desdits acides gras dans ledit diester de propylène glycol ou dans ledit triglycéride est un C8-C12 ; acide gras saturé ou insaturé.

5. Composition selon l'une quelconque des revendications 1 à 4, comprenant en outre une huile, une cire ou une combinaison de celles-ci.

6. Composition selon l'une quelconque des revendications 1 à 5, comprenant un composé de formule I, le dicaprylate de propylène glycol, le triglycéride caprylique / caprique, le diméthylsulfoxyde (DMSO), la vaseline et la cire de paraffine.

7. Composition selon l'une quelconque des revendications 1 à 6, comprenant un composé de formule I en une quantité d'environ 5 % p/p à environ 30 % p/p.

8. Composition selon la revendication 7, comprenant :
un composé de formule I en une quantité d'environ 5 % à 30 % p/p ;
le dicaprylate de propylène glycol en une quantité d'environ 1 % à environ 10 % p/p;
le triglycéride caprylique / caprique en une quantité d'environ 30 % à environ 50 % p/p ;
le diméthylsulfoxyde en une quantité d'environ 0 % à environ 10 % p/p ;
vaseline en une quantité d'environ 20 % à environ 50 % p/p ; et
de la cire de paraffine en une quantité d'environ 0 à environ 10 % p/p.

9. Composition selon la revendication 8, comprenant :
un composé de formule I en une quantité d'environ 5 % p/p ;
le dicaprylate de propylène glycol en une quantité d'environ 10 % p/p ;
le triglycéride caprylique / caprique en une quantité d'environ 50 % p/p ;
le diméthylsulfoxyde en une quantité d'environ 1 % p/p ;
vaseline en une quantité d'environ 30 % p/p ; et
de la cire de paraffine en une quantité d'environ 4 % p/p.
ou
un composé de formule I en une quantité d'environ 10 % p/p ;
le dicaprylate de propylène glycol en une quantité d'environ 7,5 % p/p ;
le triglycéride caprylique / caprique en une quantité d'environ 47,5 % p/p ;
le diméthylsulfoxyde en une quantité d'environ 1 % p/p ;
de la vaseline en une quantité d'environ 30 % p/p ; et
de la cire de paraffine en une quantité d'environ 4 % p/p ;
ou
un composé de formule I en une quantité d'environ 15 % p/p ;
le dicaprylate de propylène glycol en une quantité d'environ 5 % p/p ;
le triglycéride caprylique / caprique en une quantité d'environ 45 % p/p ;
le diméthylsulfoxyde en une quantité d'environ 1 % p/p ;
de la vaseline en une quantité d'environ 30 % p/p ; et
de la cire de paraffine en une quantité d'environ 4 % p/p.
ou
un composé de formule I en une quantité d'environ 20 % p/p ;
le dicaprylate de propylène glycol en une quantité d'environ 2,5 % p/p ;
le triglycéride caprylique / caprique en une quantité d'environ 42,5 % p/p ;
le diméthylsulfoxyde en une quantité d'environ 1 % p/p ;
de la vaseline en une quantité d'environ 30 % p/p ; et
de la cire de paraffine en une quantité d'environ 4 % p/p.

10. Composition selon l'une quelconque des revendications 1 à 9, sous la forme d'une pommade, d'une crème ou d'un gel.

11. Composition selon l'une quelconque des revendications 1 à 10, dans laquelle ladite composition est essentiellement exempte d'eau, de solvants alcooliques et d'acides carboxyliques.

12. Composition selon l'une quelconque des revendications 1 à 11, pour une utilisation dans le traitement ou la prévention du cancer, d'un état précancéreux ou d'un trouble hyperprolifératif bénin.

13. Composition à utiliser selon la revendication 12, dans laquelle ledit état ou trouble est le psoriasis ou la kératose actinique.

14. Composition à utiliser selon la revendication 12, dans laquelle ledit cancer est un cancer de la peau.

15. Composition à utiliser selon la revendication 14, dans laquelle ledit cancer de la peau comprend un mélanome ou un cancer de la peau non mélanique.

16. Composition à utiliser selon la revendication 15, dans laquelle ledit cancer de la peau sans mélanome comprend un carcinome basocellulaire (CBC), un carcinome épidermoïde (CE) ou un lymphome cutané à cellules T (LCCT).
